# EUROPEAN PATENT APPLICATION

(11) **EP 4 825 345 A1**
(43) Date of publication of application: **21.10.2026**
(21) Application number: 25904750.4
(22) Date of filing: 12.12.2025
(51) Int. Cl.: A61F 2/06

(54) **ARTIFICIAL BLOOD VESSEL AND METHOD FOR PRODUCING ARTIFICIAL BLOOD VESSEL**

(30) Priority: 13.12.2024 JP 2024219442
(71) Applicant: Tamabio Co., Ltd., Musashino-shi, Tokyo 180-0022 (JP)
(72) Inventor: NAGAO, Tetsuya, Musashino-shi, Tokyo 180-0022 (JP)
(74) Representative: Mathys & Squire
(86) International application number: PCT/JP2025/043519
(87) International publication number: WO 2026/127130

(57) **Abstract**

[Problem] Provided are an artificial blood vessel having an inner peripheral surface that favorably affects blood flow, and a method for manufacturing an artificial blood vessel having an inner peripheral surface that favorably affects blood flow.

[Solution] The artificial blood vessel comprises a tubular sheet defining a space through which blood flows. The tubular sheet includes an ion-implanted layer as an inner layer. A main component of the ion-implanted layer is polytetrafluoroethylene. An inner peripheral surface of the ion-implanted layer is constituted by a roughened surface containing polytetrafluoroethylene as a main component.

## Description

### Technical Field

The present invention relates to an artificial blood vessel and a method for manufacturing the artificial blood vessel.

### Background Art

Prosthetic materials constituting artificial blood vessels and the like are known.

As a related technique, Patent Literature 1 discloses a biological repair material having affinity for a biological tissue adhesive. Patent Literature 1 describes irradiating polytetrafluoroethylene with an ion beam in order to improve adhesion to fibrin glue.

Conventionally, irradiation of polytetrafluoroethylene with an ion beam has been performed exclusively for the purpose of improving adhesion to tissue or fibrin glue. On the other hand, utilizing an ion-irradiated surface of polytetrafluoroethylene as a surface defining a lumen through which a body fluid flows has not heretofore been performed or known. In particular, due to the physical difficulty of irradiating an inner peripheral surface of a tubular body with an ion beam, irradiation of an inner peripheral surface of an artificial blood vessel made of polytetrafluoroethylene with an ion beam has not heretofore been performed or known.

Patent Literature 2 discloses a method for manufacturing an antithrombotic material. Patent Literature 2 describes coating an inner wall of an ePTFE tube with a biopolymer material and irradiating the biopolymer material with an ion beam. However, Patent Literature 2 does not describe implanting ions into ePTFE to roughen a surface of the ePTFE.

### Citation List

### Patent Literature

[PTL1] JP 4445697 B2
[PTL2] JP H09-299474 A

### Summary of Invention

### Technical Problem

An object of the present invention is to provide an artificial blood vessel having an inner peripheral surface that favorably affects blood flow, and a method for manufacturing an artificial blood vessel having an inner peripheral surface that favorably affects blood flow.

### Solution to Problem

Embodiments of the present invention relate to the following artificial blood vessel and method for manufacturing an artificial blood vessel.
(1) An artificial blood vessel comprising a tubular sheet defining a space through which blood flows,
   wherein the tubular sheet includes an ion-implanted layer as an inner layer,
   a main component of the ion-implanted layer is polytetrafluoroethylene, and
   an inner peripheral surface of the ion-implanted layer is constituted by a roughened surface containing the polytetrafluoroethylene as a main component.
(2) The artificial blood vessel according to (1),
   wherein the ion-implanted layer is constituted by a single layer in which the polytetrafluoroethylene and an element implanted by ion implantation are mixed.
(3) The artificial blood vessel according to (1) or (2),
   wherein at least a part of the roughened surface containing the polytetrafluoroethylene as a main component is exposed to the space through which the blood flows.
(4) The artificial blood vessel according to any one of (1) to (3),
   wherein the tubular sheet is seamless.
(5) The artificial blood vessel according to any one of (1) to (4),
   wherein at least a part of an outer peripheral surface of the tubular sheet is a second roughened surface.
(6) The artificial blood vessel according to (5),
   wherein at least a part of the outer peripheral surface of the tubular sheet is a non-roughened surface.
(7) The artificial blood vessel according to any one of (1) to (6),
   wherein a drug is supported on the inner peripheral surface of the ion-implanted layer.
(8) The artificial blood vessel according to (7),
   wherein the drug includes a blood coagulation inhibitor.
(9) A method for manufacturing an artificial blood vessel, the method comprising:
   preparing a tubular sheet containing polytetrafluoroethylene; and
   irradiating the polytetrafluoroethylene with an ion beam such that an ion-implanted layer is formed in the tubular sheet,
   wherein the tubular sheet includes the ion-implanted layer as an inner layer,
   a main component of the ion-implanted layer is the polytetrafluoroethylene, and
   an inner peripheral surface of the ion-implanted layer is constituted by a roughened surface containing the polytetrafluoroethylene as a main component.
(10) The method for manufacturing an artificial blood vessel according to (9),
   wherein an angle between a direction parallel to a longitudinal direction of the tubular sheet and a direction in which the ion beam is incident on the polytetrafluoroethylene is 45 degrees or more.
(11) The method for manufacturing an artificial blood vessel according to (9) or (10), the method comprising:
   inverting the tubular sheet such that an outer surface of the tubular sheet becomes an inner surface,
   wherein irradiating the polytetrafluoroethylene with the ion beam includes irradiating the outer surface of the tubular sheet with the ion beam, and
   by the step of inverting the tubular sheet, the outer surface irradiated with the ion beam becomes the inner surface.
(12) The method for manufacturing an artificial blood vessel according to any one of (9) to (11),
   wherein, after the step of irradiating the polytetrafluoroethylene with the ion beam is performed, a layer containing a drug is added to a surface of the ion-implanted layer.
(13) The method for manufacturing an artificial blood vessel according to any one of (9) to (12), the method comprising:
   irradiating an outer peripheral surface of the tubular sheet with an ion beam such that a second ion-implanted layer is formed in the tubular sheet,
   wherein, after the step of irradiating the outer peripheral surface of the tubular sheet with the ion beam is performed, at least one of a biological adhesive and a coating for preventing leakage of blood is added to a surface of the second ion-implanted layer.

### Advantageous Effects of Invention

According to the present invention, it is possible to provide an artificial blood vessel having an inner peripheral surface that favorably affects blood flow, and a method for manufacturing an artificial blood vessel having an inner peripheral surface that favorably affects blood flow.

### Brief Description of Drawings

FIG. 1 is a schematic perspective view schematically illustrating an artificial blood vessel according to a first embodiment.
FIG. 2 is a schematic cross-sectional view schematically illustrating the artificial blood vessel according to the first embodiment.
FIG. 3 is a schematic cross-sectional view schematically illustrating a state in which the artificial blood vessel according to the first embodiment is attached to a first blood vessel and a second blood vessel.
FIG. 4 is a schematic cross-sectional view schematically illustrating a state in which tissue of a patient has extended over an inner peripheral surface of an ion-implanted layer.
FIG. 5 is a schematic cross-sectional view schematically illustrating a state in which an inner peripheral surface of an end portion of the artificial blood vessel according to the first embodiment is attached to an outer peripheral surface of an end portion of a blood vessel of a patient.
FIG. 6 is a schematic cross-sectional view schematically illustrating a state in which the artificial blood vessel is attached to a blood vessel of a patient, with an end surface of the artificial blood vessel according to the first embodiment being in contact with an end surface of the blood vessel of the patient.
FIG. 7 is a schematic perspective view schematically illustrating an artificial blood vessel according to a first modification of the first embodiment.
FIG. 8 is a schematic cross-sectional view schematically illustrating a state in which the artificial blood vessel according to the first modification of the first embodiment is attached to a first blood vessel and a second blood vessel.
FIG. 9 is a schematic perspective view schematically illustrating an artificial blood vessel according to a second modification of the first embodiment.
FIG. 10 is a schematic cross-sectional view schematically illustrating a state in which the artificial blood vessel according to the second modification of the first embodiment is attached to a first blood vessel and a second blood vessel.
FIG. 11 is a schematic cross-sectional view schematically illustrating a state in which the artificial blood vessel according to the first embodiment supports a drug.
FIG. 12 is a schematic cross-sectional view schematically illustrating a state in which an artificial blood vessel according to a modification of the first embodiment supports a drug.
FIG. 13 is a schematic perspective view schematically illustrating an example of a tubular sheet prepared in a preparation step.
FIG. 14 is a schematic cross-sectional view schematically illustrating a manner in which an ion beam is emitted from an ion irradiation apparatus onto an inner peripheral surface of the tubular sheet.
FIG. 15 is a schematic cross-sectional view schematically illustrating a manner in which an ion beam is emitted from an ion irradiation apparatus onto an outer surface of the tubular sheet.
FIG. 16 is a diagram schematically illustrating a manner in which the tubular sheet is inverted.
FIG. 17 is a schematic perspective view schematically illustrating a state during execution of an inversion step.
FIG. 18 is a schematic cross-sectional view schematically illustrating a state during execution of a second irradiation step.
FIG. 19 is a schematic cross-sectional view schematically illustrating a manner in which an ion beam is emitted onto only a part of an outer peripheral surface of the tubular sheet.
FIG. 20 is a schematic cross-sectional view schematically illustrating a state in which, after execution of the second irradiation step, a biological adhesive has been added to a surface of a second ion-implanted layer.
FIG. 21 is a schematic cross-sectional view schematically illustrating a state in which, after execution of the second irradiation step, a coating for preventing leakage of blood has been added to a surface of a second ion-implanted layer.
FIG. 22 is a schematic cross-sectional view schematically illustrating a manner in which an ion beam is emitted onto an end surface of the tubular sheet on a first direction side.
FIG. 23 is a schematic cross-sectional view schematically illustrating a manner in which an ion beam is emitted onto an end surface of the tubular sheet on a second direction side.
FIG. 24 is a flowchart illustrating an example of a method for manufacturing an artificial blood vessel according to a second embodiment.

### Description of Embodiments

Hereinafter, an artificial blood vessel 1A according to an embodiment and a method for manufacturing the artificial blood vessel 1 will be described with reference to the drawings. In the following description, members and portions having the same functions are denoted by the same reference signs, and repeated descriptions of the members and portions denoted by the same reference signs are omitted.

### Definitions of Terms

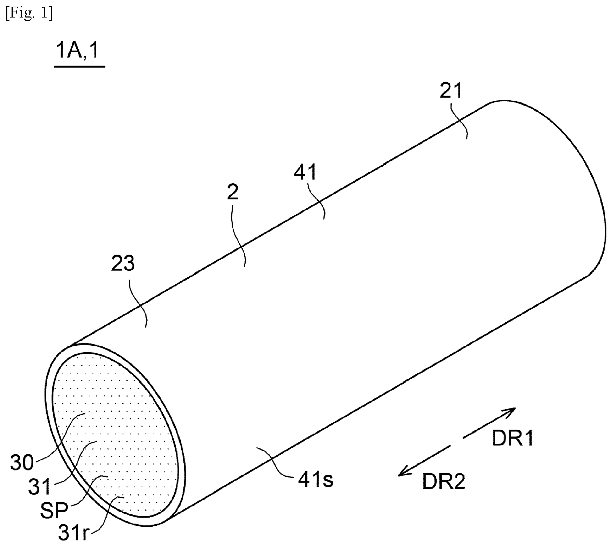

In the present specification, a direction from a second end portion 23 of a tubular sheet 2 toward a first end portion 21 of the tubular sheet 2 is defined as a first direction DR1. A direction opposite to the first direction DR1 is defined as a second direction DR2. In the example illustrated in FIG. 1, each of the first direction DR1 and the second direction DR2 coincides with a longitudinal direction of the tubular sheet 2.

### First Embodiment

An artificial blood vessel 1A according to a first embodiment will be described with reference to FIGS. 1 to 12.

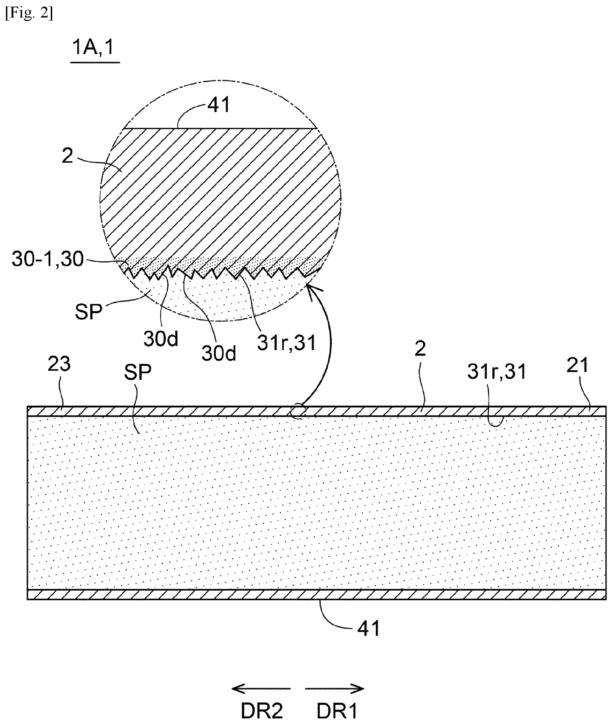

FIG. 1 is a schematic perspective view schematically illustrating the artificial blood vessel 1A according to the first embodiment. FIG. 2 is a schematic cross-sectional view schematically illustrating the artificial blood vessel 1A according to the first embodiment.

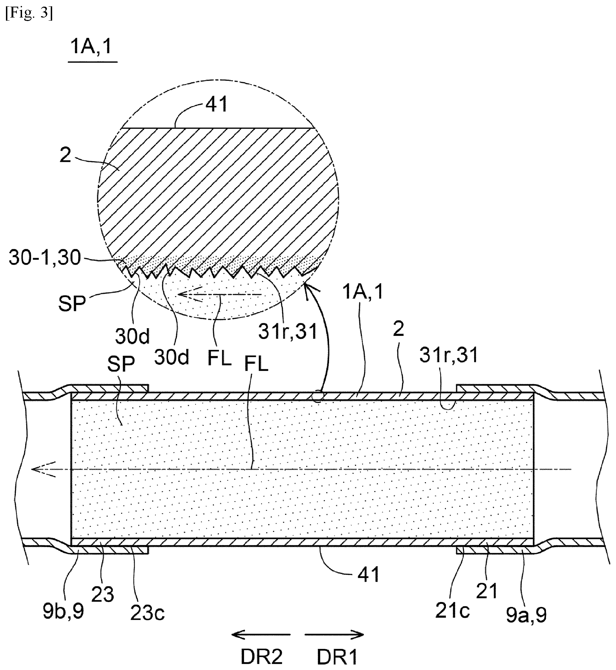

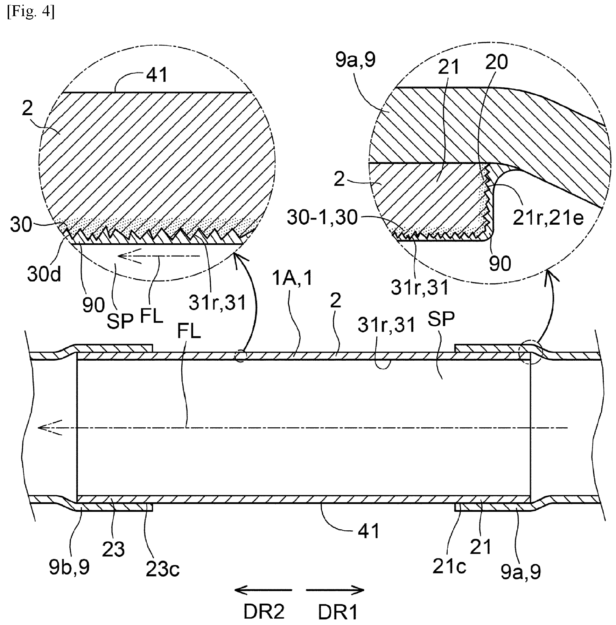

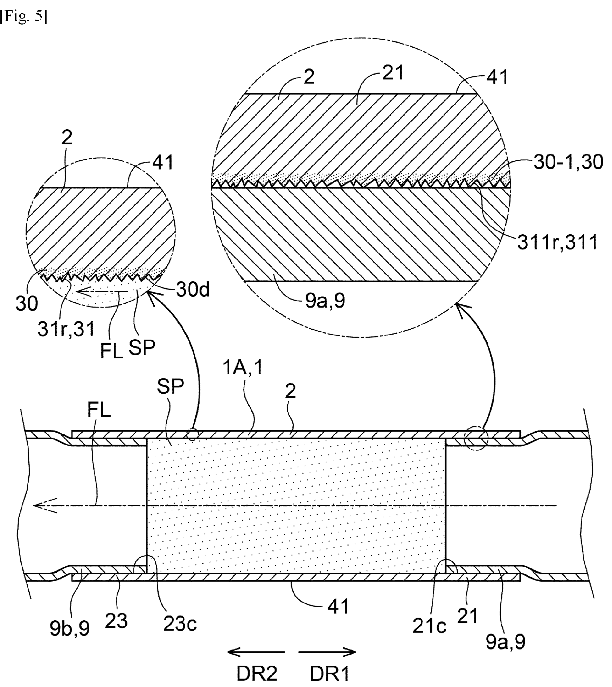

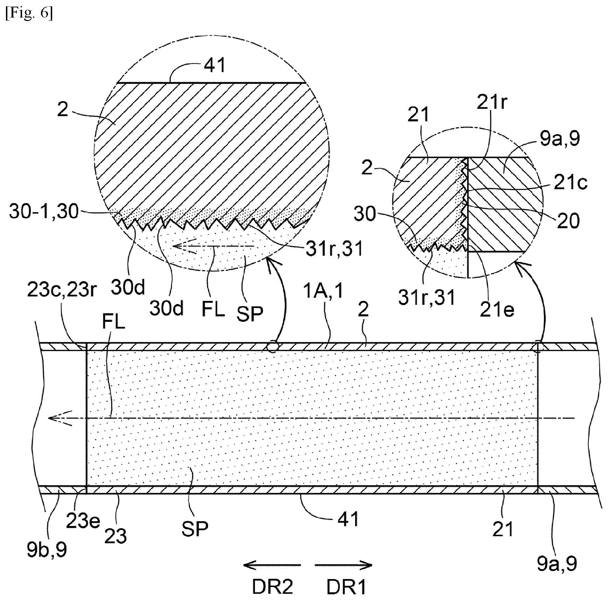

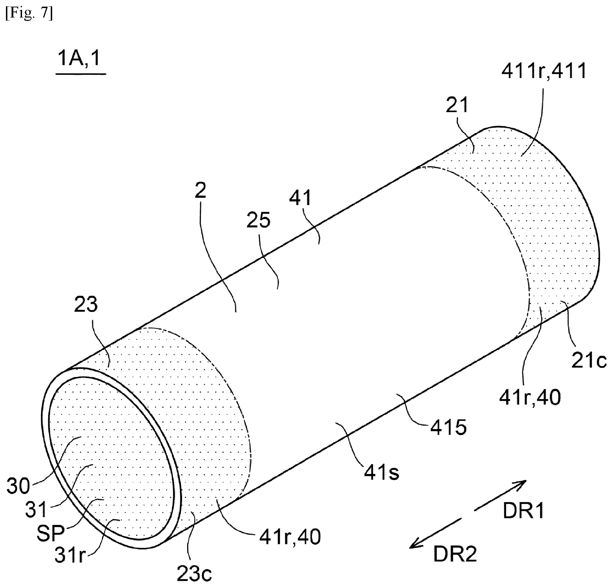

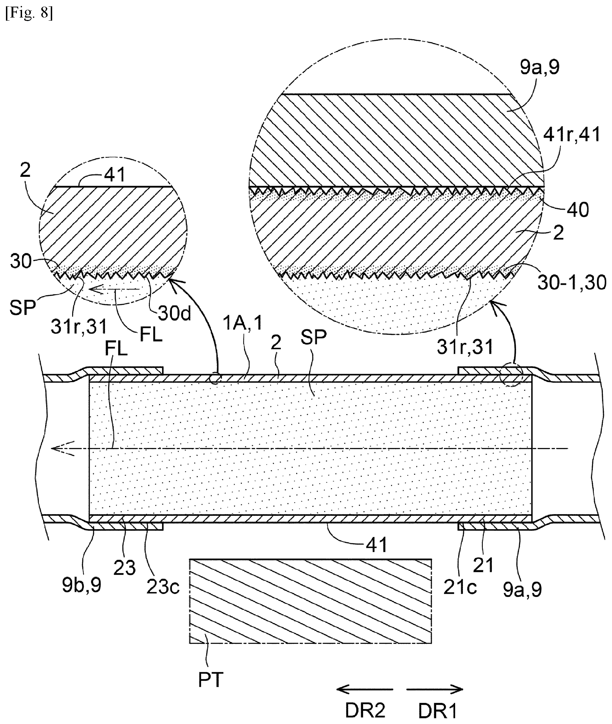

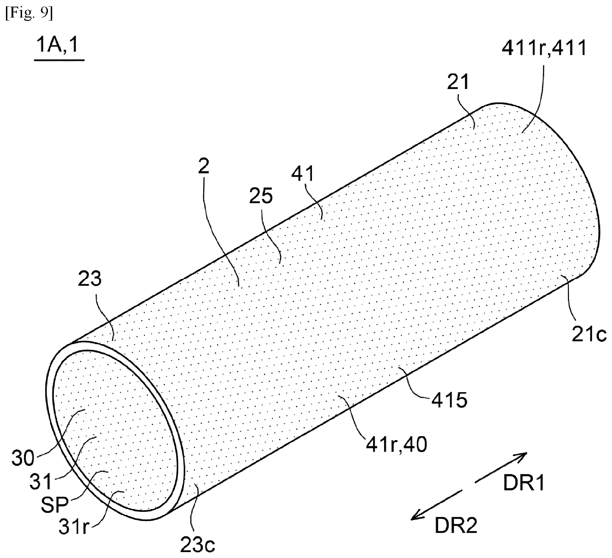

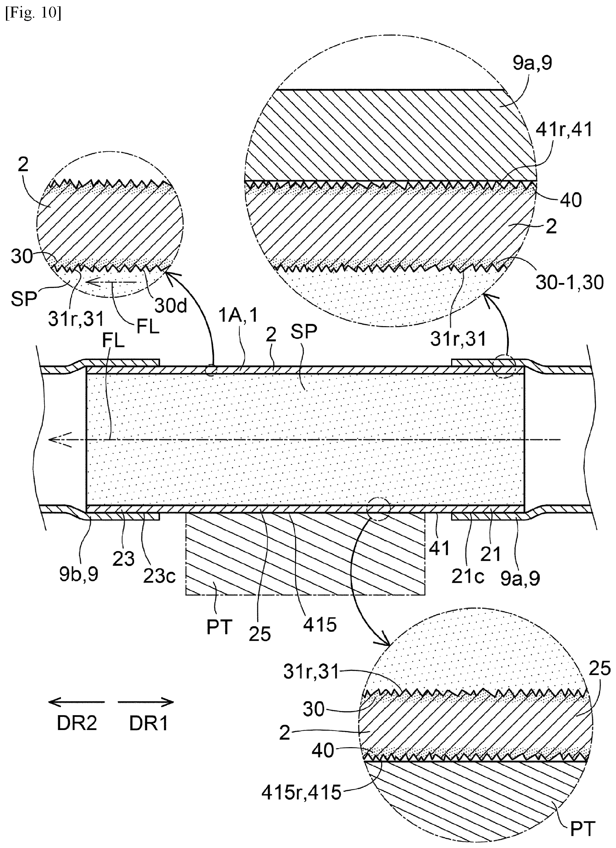

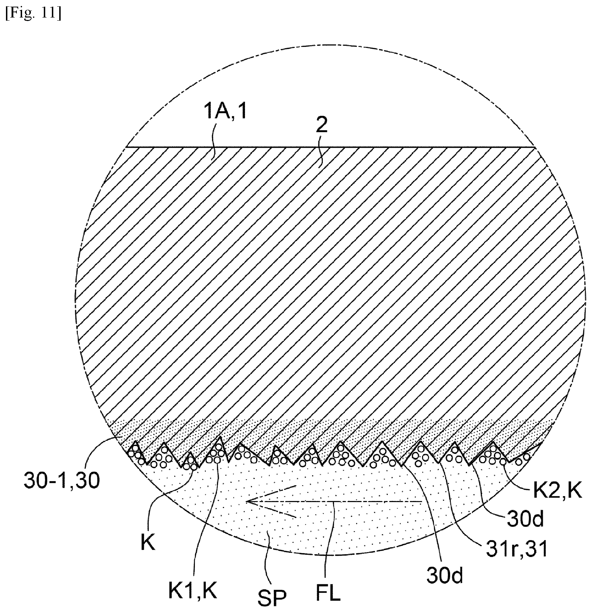

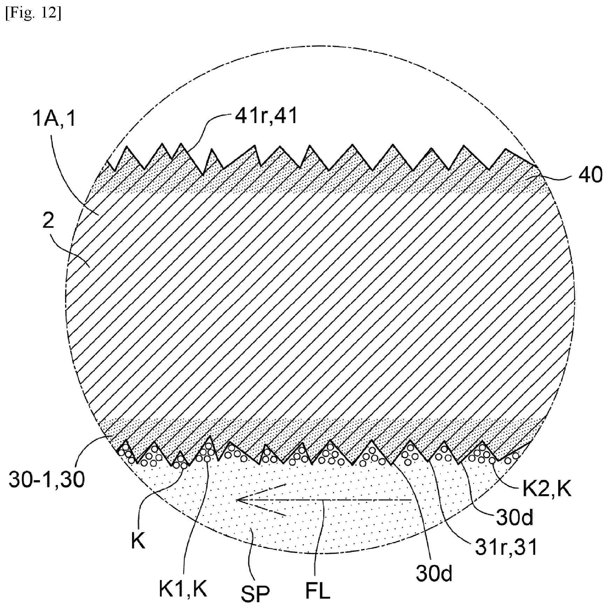

FIG. 3 is a schematic cross-sectional view schematically illustrating a state in which the artificial blood vessel 1A according to the first embodiment is attached to a first blood vessel 9a and a second blood vessel 9b. FIG. 4 is a schematic cross-sectional view schematically illustrating a state in which tissue 90 of a patient has extended over an inner peripheral surface 31 of an ion-implanted layer 30. FIG. 5 is a schematic cross-sectional view schematically illustrating a state in which an inner peripheral surface of an end portion of the artificial blood vessel 1A according to the first embodiment is attached to an outer peripheral surface of an end portion of a blood vessel 9 of a patient. FIG. 6 is a schematic cross-sectional view schematically illustrating a state in which the artificial blood vessel 1A is attached to the blood vessel 9 of the patient, with an end surface of the artificial blood vessel 1A according to the first embodiment being in contact with an end surface of the blood vessel 9 of the patient. FIG. 7 is a schematic perspective view schematically illustrating an artificial blood vessel 1A according to a first modification of the first embodiment. FIG. 8 is a schematic cross-sectional view schematically illustrating a state in which the artificial blood vessel 1A according to the first modification of the first embodiment is attached to the first blood vessel 9a and the second blood vessel 9b. FIG. 9 is a schematic perspective view schematically illustrating an artificial blood vessel 1A according to a second modification of the first embodiment. FIG. 10 is a schematic cross-sectional view schematically illustrating a state in which the artificial blood vessel 1A according to the second modification of the first embodiment is attached to the first blood vessel 9a and the second blood vessel 9b. FIG. 11 is a schematic cross-sectional view schematically illustrating a state in which the artificial blood vessel 1A according to the first embodiment supports a drug K. FIG. 12 is a schematic cross-sectional view schematically illustrating a state in which an artificial blood vessel 1A according to a modification of the first embodiment supports a drug K.

As illustrated in FIG. 1, the artificial blood vessel 1A according to the first embodiment includes a tubular sheet 2. The tubular sheet 2 defines a space SP through which blood flows.

As illustrated in FIG. 2, the tubular sheet 2 includes an ion-implanted layer 30 as an inner layer. A main component of the ion-implanted layer 30 is polytetrafluoroethylene.

In the example illustrated in FIG. 2, the inner peripheral surface 31 of the ion-implanted layer 30 is constituted by a roughened surface 31r containing polytetrafluoroethylene as a main component. In other words, a proportion of polytetrafluoroethylene constituting the roughened surface in the entire material constituting the roughened surface 31r is 50 percent by weight or more. The proportion of polytetrafluoroethylene constituting the roughened surface in the entire material constituting the roughened surface 31r may be 70 percent by weight or more, 90 percent by weight or more, 95 percent by weight or more, or 99 percent by weight or more.

The entirety of the tubular sheet 2 may contain polytetrafluoroethylene as a main component. In other words, polytetrafluoroethylene may account for 50 percent by weight or more of the entire material constituting the tubular sheet 2. Polytetrafluoroethylene may account for 70 percent by weight or more, 90 percent by weight or more, 95 percent by weight or more, or 99 percent by weight or more of the entire material constituting the tubular sheet 2. Alternatively, the tubular sheet 2 may be a laminate including an inner layer constituted by polytetrafluoroethylene and an outer layer constituted by another material.

In the example illustrated in FIG. 2, the roughened surface 31r is constituted by the inner peripheral surface 31 of the ion-implanted layer 30. In the present specification, the term "ion-implanted layer 30" means a layer whose physical and/or chemical properties have been modified by implantation of an ionized element. In the example illustrated in FIG. 2, the ion-implanted layer 30 has a plurality of fine recesses 30d in its surface due to ion implantation.

The roughened surface 31r containing polytetrafluoroethylene as a main component, more specifically, the inner peripheral surface 31 of the ion-implanted layer 30, favorably affects blood flow. More specifically, the roughened surface 31r containing polytetrafluoroethylene as a main component, more specifically, the inner peripheral surface 31 of the ion-implanted layer 30, reduces frictional resistance between an inner surface of the tubular sheet 2 and blood flowing through the tubular sheet 2, similarly to a riblet. In this manner, blood flow FL of a patient to whom the artificial blood vessel 1A is applied is improved. See FIG. 3. A degree of roughening of the roughened surface 31r may be set such that a Reynolds number of blood flowing through the tubular sheet 2 has an appropriate value. For example, the degree of roughening of the roughened surface 31r may be set such that blood flow in the vicinity of the inner peripheral surface 31 becomes laminar.

The roughened surface 31r containing polytetrafluoroethylene as a main component allows cells of a patient to extend more easily than a non-roughened surface. Accordingly, as illustrated in FIG. 4, after the artificial blood vessel 1A is anastomosed to a blood vessel 9 of the patient, the roughened surface 31r of the tubular sheet 2 is covered with tissue 90 of the patient, more specifically, cells of the patient, relatively quickly. After the roughened surface 31r of the tubular sheet 2 is covered with the tissue 90 of the patient, the tubular sheet 2 is expected to act on blood flow like the blood vessel 9 of the patient.

### Optional Additional Configurations

Next, optional additional configurations that can be employed in the artificial blood vessel 1A according to the first embodiment will be described.

### Expanded Polytetrafluoroethylene

In the present specification, the polytetrafluoroethylene is preferably expanded polytetrafluoroethylene, hereinafter referred to as "ePTFE." The ePTFE is polytetrafluoroethylene that has been subjected to an expansion treatment, more specifically, polytetrafluoroethylene that has been expanded in a heated state. The ePTEF is, for example, expanded porous polytetrafluoroethylene produced using a method described in U.S. Patent No. 3,953,566 or 4,187,390. Alternatively, the polytetrafluoroethylene in the present specification may be non-expanded polytetrafluoroethylene.

### Ion-Implanted Layer 30

The ion-implanted layer 30 is a layer in which an ion-implanted element is mixed with polytetrafluoroethylene. A part of the ion-implanted element may be released from the ion-implanted layer 30. The element ion-implanted into the polytetrafluoroethylene may be argon, neon, or another element. In other words, the ion-implanted layer 30 may be a layer in which argon is mixed with polytetrafluoroethylene, a layer in which neon is mixed with polytetrafluoroethylene, or a layer in which another element is mixed with polytetrafluoroethylene.

As illustrated in FIG. 2, the ion-implanted layer 30 may be constituted by a single layer 30-1 in which an element, for example, argon or neon, implanted by ion implantation is mixed with polytetrafluoroethylene.

As illustrated in FIG. 2, at least a part of the roughened surface 31r containing polytetrafluoroethylene as a main component is exposed to the space SP through which blood flows. Substantially the entirety of the roughened surface 31r containing polytetrafluoroethylene as a main component may be exposed to the space SP through which blood flows. The roughened surface 31r favorably affects blood flow. In addition, the roughened surface 31r allows cells of a patient, more specifically, endothelial cells, to extend more easily than a non-roughened surface.

Substantially the entirety of the inner surface of the tubular sheet 2 may be the roughened surface 31r, more specifically, the inner peripheral surface 31 of the ion-implanted layer 30. In this case, the inner peripheral surface 31 of the ion-implanted layer 30 as a whole contributes to reducing frictional resistance against blood flow. In addition, tissue 90 of a patient, more specifically, endothelial cells of the patient, can easily extend over the entirety of the inner peripheral surface 31 of the ion-implanted layer 30.

A maximum height roughness Rz of the inner peripheral surface 31 of the ion-implanted layer 30 is, for example, 8 µm or more. In the present specification, the "maximum height roughness Rz" is measured based on JIS B 0601:2013, corresponding to International Standard ISO 4287:1997, Amd. 1:2009.

### Seamless Tubular Sheet 2

The tubular sheet 2 is preferably seamless. In this case, since the inner surface of the tubular sheet 2 is seamless, blood flow is made smoother. In the example illustrated in FIG. 1, the tubular sheet 2 is a seamless tubular sheet.

The seamless tubular sheet 2 is formed, for example, by extrusion molding. More specifically, the seamless tubular sheet 2 is formed by extrusion-molding a raw material containing polytetrafluoroethylene as a main component into a tubular shape. In contrast, when the tubular sheet 2 is formed by rolling a flat sheet, the presence of a joint, in other words, a seam, is unavoidable.

After the tubular sheet 2 is formed, an outer surface of the tubular sheet 2 may be roughened by ion irradiation. After the outer surface of the tubular sheet 2 is roughened, the tubular sheet 2 may be inverted such that the outer surface of the tubular sheet 2 becomes an inner surface. By the inversion, the inner surface of the tubular sheet 2 becomes the roughened surface 31r. By the inversion, technical difficulty in making an inner layer of the tubular sheet 2 an ion-implanted layer is overcome.

### Tubular Sheet 2

The artificial blood vessel 1A according to the first embodiment includes the tubular sheet 2. More specifically, the artificial blood vessel 1A is constituted by the tubular sheet 2. A film thickness of the tubular sheet 2 is, for example, 0.01 mm or more and 2 mm or less. The film thickness of the tubular sheet 2 may be 0.05 mm or more and 1.5 mm or less, 0.1 mm or more and 1.0 mm or less, or 0.1 mm or more and 0.5 mm or less.

### First End Portion 21 of Tubular Sheet 2

In the example illustrated in FIG. 3, the tubular sheet 2 has a first end portion 21 attached to a first blood vessel 9a of a patient. In the example illustrated in FIG. 3, the first end portion 21 is attached to an end portion of the first blood vessel 9a of the patient by end-to-end anastomosis. Alternatively, the first end portion 21 may be attached to the first blood vessel 9a of the patient by end-to-side anastomosis. The first end portion 21 may be attached to the first blood vessel 9a of the patient by suturing. Alternatively or additionally, the first end portion 21 may be attached to the first blood vessel 9a of the patient by a biological adhesive, for example, fibrin glue. Any known method can be employed as a method for attaching the first end portion 21 to the first blood vessel 9a.

A first contact layer 21c of the first end portion 21 that is to come into contact with the first blood vessel 9a preferably includes a roughened surface, more specifically, a surface of an ion-implanted layer. See FIGS. 7 and 8. In this case, cells of the patient can easily extend between the first contact layer 21c and the first blood vessel 9a. Accordingly, blood is less likely to leak from between the first contact layer 21c and the first blood vessel 9a.
A biological adhesive may be applied to the first contact layer 21c. When the first contact layer 21c includes a roughened surface, more specifically, a surface of an ion-implanted layer, the roughened surface favorably supports the biological adhesive. The first contact layer 21c may be a layer in which a coating for preventing leakage of blood is applied to the roughened surface, more specifically, the surface of the ion-implanted layer.

As illustrated in FIG. 3, the first end portion 21 of the tubular sheet 2 may be configured to be attached to the first blood vessel 9a in a state in which the first end portion 21 is inserted inside an end portion of the first blood vessel 9a. Alternatively, as illustrated in FIG. 5, the first end portion 21 of the tubular sheet 2 may be configured to be attached to the first blood vessel 9a in a state in which an end portion of the first blood vessel 9a is inserted inside the first end portion 21 of the tubular sheet 2.

Further alternatively, as illustrated in FIG. 6, the first end portion 21 of the tubular sheet 2 may be configured to be attached to the first blood vessel 9a in a state in which an end surface 21e of the first end portion 21 of the tubular sheet 2 is in contact with an end surface of an end portion of the first blood vessel 9a.

In the example illustrated in FIG. 6, the end surface 21e of the first end portion 21 of the tubular sheet 2, more specifically, the end surface 21e of the tubular sheet 2 on the first direction DR1 side, includes a roughened surface 21r. In the example illustrated in FIG. 6, the roughened surface 21r is constituted by a surface of an ion-implanted layer 20. When the end surface 21e includes the roughened surface 21r, more specifically, the surface of the ion-implanted layer 20, cells of the patient rapidly extend over the roughened surface 21r. Accordingly, leakage of blood from a gap between the end surface 21e and the end surface of the first blood vessel 9a is prevented. From the viewpoint of preventing blood from leaking from the gap before cells of the patient extend over the roughened surface 21r, more specifically, the surface of the ion-implanted layer 20, a biological adhesive may be applied to the roughened surface 21r.

As illustrated in FIG. 4, even when the first end portion 21 of the tubular sheet 2 is inserted inside an end portion of the first blood vessel 9a, tissue 90 of the patient, more specifically, cells of the patient, rapidly extend over the roughened surface 21r of the first end portion 21 on the first direction DR1 side, more specifically, the surface of the ion-implanted layer 20. Accordingly, leakage of blood is prevented, and blood flow in the vicinity of the end surface 21e of the tubular sheet 2 on the first direction DR1 side is improved.

### Second End Portion 23 of Tubular Sheet 2

In the example illustrated in FIG. 3, the tubular sheet 2 has a second end portion 23 attached to a second blood vessel 9b of a patient. In the example illustrated in FIG. 3, the second end portion 23 is attached to an end portion of the second blood vessel 9b of the patient by end-to-end anastomosis. Alternatively, the second end portion 23 may be attached to the second blood vessel 9b of the patient by end-to-side anastomosis. The second end portion 23 may be attached to the second blood vessel 9b of the patient by suturing. Alternatively or additionally, the second end portion 23 may be attached to the second blood vessel 9b of the patient by a biological adhesive, for example, fibrin glue. Any known method can be employed as a method for attaching the second end portion 23 to the second blood vessel 9b.

A second contact layer 23c of the second end portion 23 that is to come into contact with the second blood vessel 9b preferably includes a roughened surface, more specifically, a surface of an ion-implanted layer. See FIGS. 7 and 8. In this case, cells of the patient can easily extend between the second contact layer 23c and the second blood vessel 9b. Accordingly, blood is less likely to leak from between the second contact layer 23c and the second blood vessel 9b. A biological adhesive may be applied to the second contact layer 23c. When the second contact layer 23c includes a roughened surface, more specifically, a surface of an ion-implanted layer, the second contact layer 23c favorably supports the biological adhesive. The second contact layer 23c may be a layer in which a coating for preventing leakage of blood is applied to the roughened surface, more specifically, the surface of the ion-implanted layer.

As illustrated in FIG. 3, the second end portion 23 of the tubular sheet 2 may be configured to be attached to the second blood vessel 9b in a state in which the second end portion 23 is inserted inside an end portion of the second blood vessel 9b. Alternatively, as illustrated in FIG. 5, the second end portion 23 of the tubular sheet 2 may be configured to be attached to the second blood vessel 9b in a state in which an end portion of the second blood vessel 9b is inserted inside the second end portion 23 of the tubular sheet 2.

Further alternatively, as illustrated in FIG. 6, the second end portion 23 of the tubular sheet 2 may be configured to be attached to the second blood vessel 9b in a state in which an end surface 23e of the second end portion 23 of the tubular sheet 2 is in contact with an end surface of an end portion of the second blood vessel 9b.

An end surface 23e of the second end portion 23 of the tubular sheet 2, more specifically, the end surface 23e of the tubular sheet 2 on the second direction DR2 side, may include a roughened surface 23r. The roughened surface 23r may be constituted by a surface of an ion-implanted layer.

### Outer Peripheral Surface 41 of Tubular Sheet 2

As illustrated in FIG. 1, an outer peripheral surface 41 of the tubular sheet 2 may be a non-roughened surface 41s, more specifically, a smooth surface. In the example illustrated in FIG. 1, substantially the entirety of the outer peripheral surface 41 of the tubular sheet 2 is the non-roughened surface 41s, more specifically, a smooth surface. In the present specification, the term "non-roughened surface" means a surface that has not been subjected to a roughening treatment and that has a maximum height roughness Rz smaller than that of the roughened surface 31r.

In the examples illustrated in FIGS. 1 and 7, at least a part of the outer peripheral surface 41 of the tubular sheet 2, for example, an outer peripheral surface 415 of a longitudinal central region 25 of the tubular sheet 2, is the non-roughened surface 41s. The non-roughened surface 41s is less likely to adhere to living tissue of the patient.

In the examples illustrated in FIGS. 7 and 9, at least a part of the outer peripheral surface 41 of the tubular sheet 2 is a roughened surface 41r. The outer peripheral surface 41 of the tubular sheet 2 preferably includes the roughened surface 41r containing polytetrafluoroethylene as a main component. Substantially the entirety of the outer peripheral surface 41 of the tubular sheet 2 may contain polytetrafluoroethylene as a main component.

As illustrated in FIGS. 8 and 10, the roughened surface 41r is constituted by a surface of an ion-implanted layer 40. The ion-implanted layer 40 is, for example, a layer in which an ion-implanted element is mixed with polytetrafluoroethylene. The element implanted into the tubular sheet 2, more specifically, the element ion-implanted into the polytetrafluoroethylene, is, for example, argon, neon, or the like. The ion-implanted layer 40 may be a layer in which argon is mixed with polytetrafluoroethylene, or may be a layer in which neon is mixed with polytetrafluoroethylene.

A maximum height roughness Rz of the roughened surface 41r is, for example, 8 µm or more.

As illustrated in FIGS. 7 and 9, an outer peripheral surface 411 of the first end portion 21 of the tubular sheet 2 may be a roughened surface 411r. The roughened surface 411r is constituted by a surface of the ion-implanted layer 40.

When the outer peripheral surface 411 of the first end portion 21 of the tubular sheet 2 is the roughened surface 411r, more specifically, the surface of the ion-implanted layer 40, cells of the patient can easily extend between the outer peripheral surface 411 and the first blood vessel 9a after the outer peripheral surface 411 of the first end portion 21 is attached to the first blood vessel 9a. Accordingly, blood is less likely to leak from between the outer peripheral surface 411 and the first blood vessel 9a. Alternatively or additionally, as illustrated in FIG. 5, an inner peripheral surface 311 of the first end portion 21 of the tubular sheet 2 may be a roughened surface 311r, more specifically, a surface of the ion-implanted layer 30. In this case, cells of the patient can easily extend between the inner peripheral surface 311 and the first blood vessel 9a after the inner peripheral surface 311 of the first end portion 21 is attached to the first blood vessel 9a. Accordingly, blood is less likely to leak from between the inner peripheral surface 311 and the first blood vessel 9a.

As illustrated in FIG. 10, an outer peripheral surface 415 of a longitudinal central region 25 of the tubular sheet 2 may be a roughened surface 415r, more specifically, a surface of the ion-implanted layer 40. In this case, the outer peripheral surface 415 of the longitudinal central region 25 of the tubular sheet 2 may be fixed to living tissue PT of the patient. The fixing may be performed by allowing tissue of the patient, more specifically, cells of the patient, to extend over the roughened surface 415r through contact between the roughened surface 415r and the living tissue PT. Alternatively or additionally, the fixing may be performed using a biological adhesive, suturing, or the like.

As illustrated in FIG. 9, substantially the entirety of the outer peripheral surface 41 of the tubular sheet 2 may be the roughened surface 41r, more specifically, the surface of the ion-implanted layer 40.

In the present specification, in order to terminologically distinguish the "roughened surface 31r" included in the inner layer of the tubular sheet 2 from the "roughened surface 41r" included in the outer layer of the tubular sheet 2, the latter is referred to as a "second roughened surface 41r." In the present specification, in order to terminologically distinguish the "roughened surface 31r" included in the inner layer of the tubular sheet 2 from the "roughened surface 21r" included in the end surface 21e of the first end portion 21 of the tubular sheet 2, the latter is referred to as a "third roughened surface 21r." In the present specification, in order to terminologically distinguish the "roughened surface 31r" included in the inner layer of the tubular sheet 2 from the "roughened surface 23r" included in the end surface 23e of the second end portion 23 of the tubular sheet 2, the latter is referred to as a "fourth roughened surface 23r."

In the present specification, in order to terminologically distinguish the "ion-implanted layer 30" on the inner surface side of the tubular sheet 2 from the "ion-implanted layer 40" on the outer surface side of the tubular sheet 2, the latter is referred to as a "second ion-implanted layer 40." In the present specification, in order to terminologically distinguish the "ion-implanted layer 30" on the inner surface side of the tubular sheet 2 from the "ion-implanted layer 20" on the end surface 21e side in the first direction DR1 of the tubular sheet 2, the latter is referred to as a "third ion-implanted layer 20."

The surface roughness of the roughened surface 31r, more specifically, the maximum height roughness Rz, may be the same as the surface roughness of the second roughened surface 41r, more specifically, the maximum height roughness Rz. Alternatively, the surface roughness of the roughened surface 31r, more specifically, the maximum height roughness Rz, may be different from the surface roughness of the second roughened surface 41r, more specifically, the maximum height roughness Rz. A function of the roughened surface 31r, for example, a function of favorably affecting blood flow, and a function of the second roughened surface 41r, for example, a function of improving adhesion to patient tissue, are different from each other. Accordingly, a difference between the surface roughness of the roughened surface 31r, more specifically, the maximum height roughness Rz, and the surface roughness of the second roughened surface 41r, more specifically, the maximum height roughness Rz, may be set in accordance with the difference between the functions thereof.

The surface roughness of the roughened surface 31r, more specifically, the maximum height roughness Rz, may be the same as the surface roughness of the third roughened surface 21r, more specifically, the maximum height roughness Rz. Alternatively, the surface roughness of the roughened surface 31r, more specifically, the maximum height roughness Rz, may be different from the surface roughness of the third roughened surface 21r, more specifically, the maximum height roughness Rz.

### Drug K

As illustrated in FIGS. 11 and 12, a drug K may be supported on the inner peripheral surface 31 of the ion-implanted layer 30. A plurality of fine recesses 30d are formed in the inner peripheral surface 31 of the ion-implanted layer 30 by ion implantation. The recesses 30d favorably support the drug K.

A drug that suppresses coagulation of blood, hereinafter referred to as a "blood coagulation inhibitor K1," may be supported on the inner peripheral surface 31 of the ion-implanted layer 30. In other words, the drug K supported by the inner peripheral surface 31 of the ion-implanted layer 30 may include the blood coagulation inhibitor K1, for example, an anticoagulant or an antiplatelet drug. Since blood coagulation inhibitors are known, descriptions of blood coagulation inhibitors are omitted.

Alternatively or additionally, a drug K2 that regulates extension of vascular endothelial cells may be supported on the inner peripheral surface 31 of the ion-implanted layer 30. The drug K2 that regulates extension of vascular endothelial cells may be a drug that stimulates proliferation of vascular endothelial cells, for example, a drug containing vascular endothelial growth factor (VEGF), or may be a cytostatic agent that suppresses proliferation of vascular endothelial cells. By the drug K2 that regulates extension of vascular endothelial cells, a period until the inner surface of the tubular sheet 2 is covered with tissue of the patient, more specifically, endothelial cells of the patient, can be suitably controlled.

In the examples illustrated in FIGS. 11 and 12, a part of the inner peripheral surface 31 of the ion-implanted layer 30 is exposed to the space SP through which blood flows without being covered with the drug K. Alternatively, the entirety of the inner peripheral surface 31 of the ion-implanted layer 30 may be covered with the drug K. In these cases, the inner peripheral surface 31 of the ion-implanted layer 30 is configured to be exposed to the space SP through which blood flows as the drug K is gradually released into the blood. After the inner peripheral surface 31 of the ion-implanted layer 30 is exposed to the space SP through which blood flows, the inner peripheral surface 31 contributes to reducing frictional resistance against blood flow. In addition, tissue 90 of the patient, more specifically, endothelial cells of the patient, extends over the inner peripheral surface 31 of the ion-implanted layer 30.

### Second Embodiment

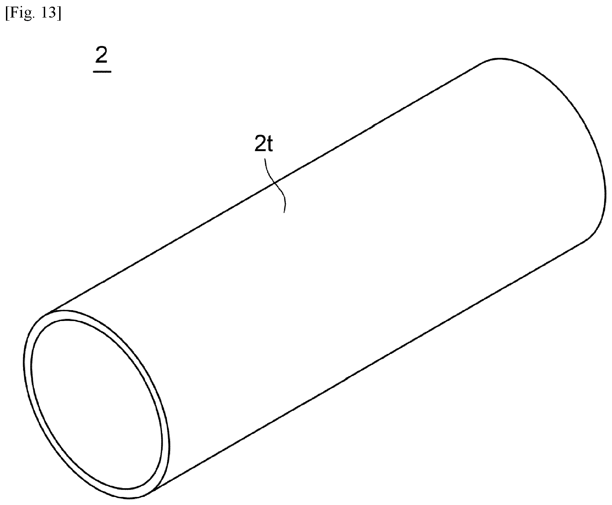

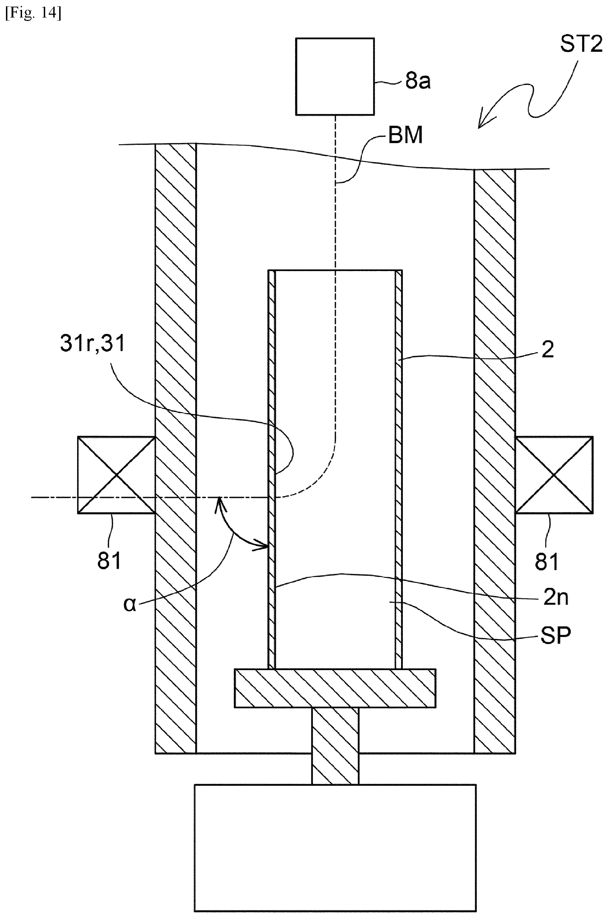

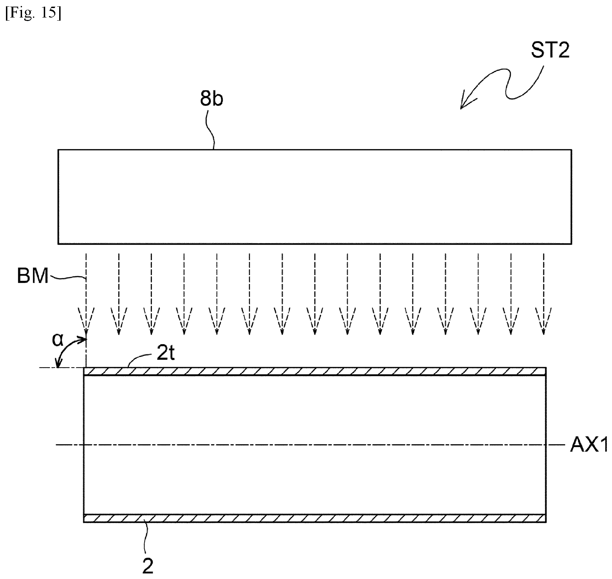

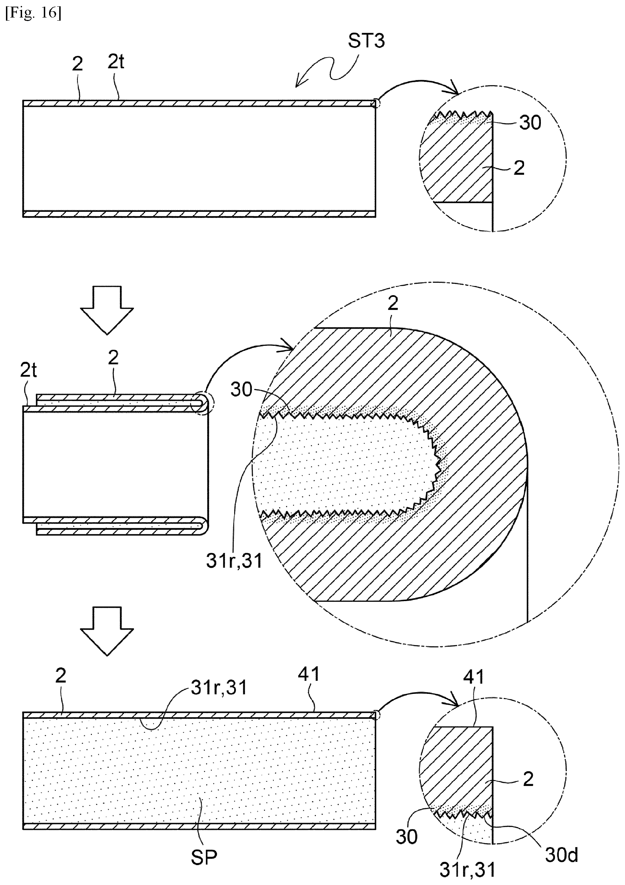

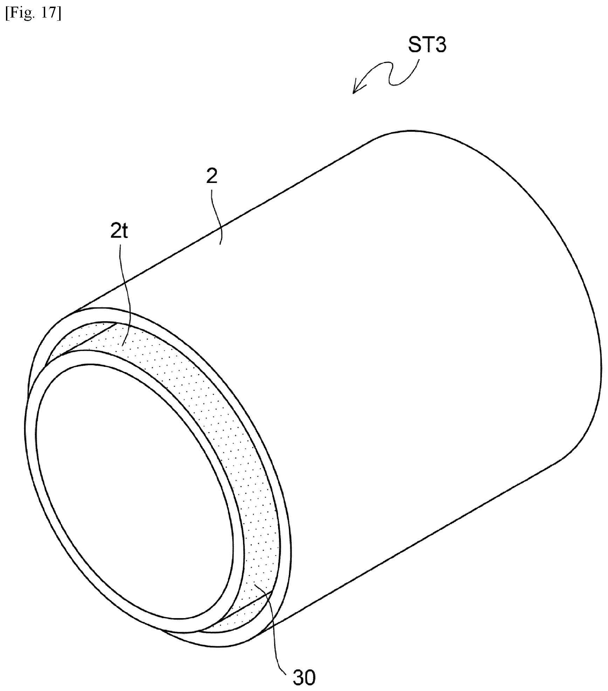

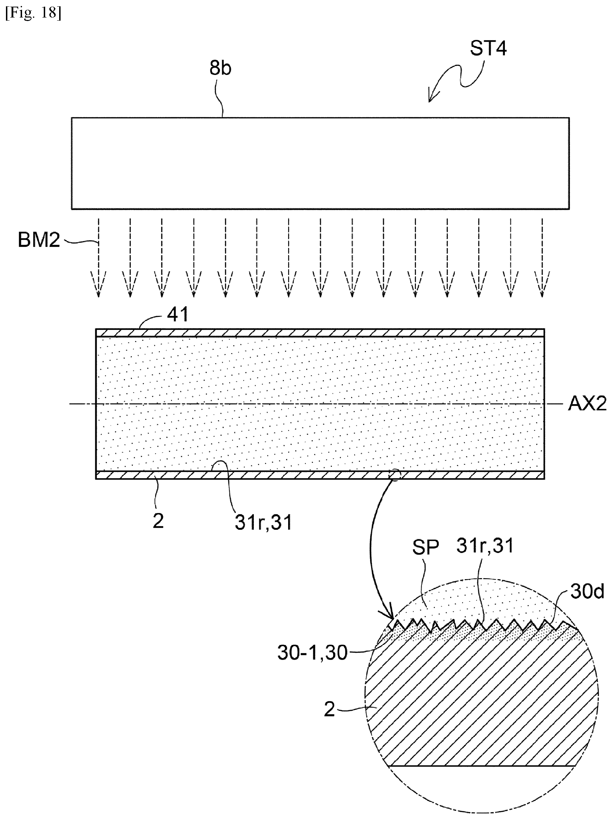

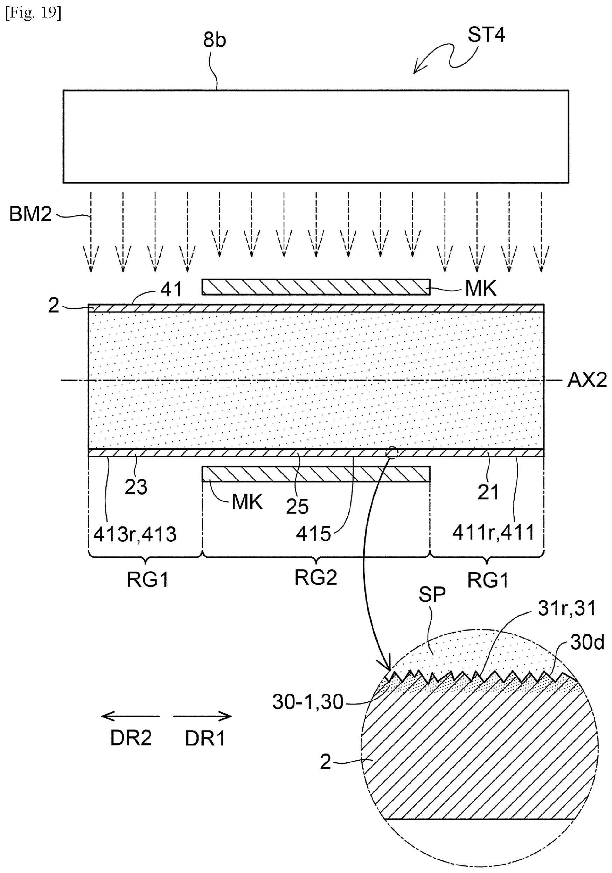

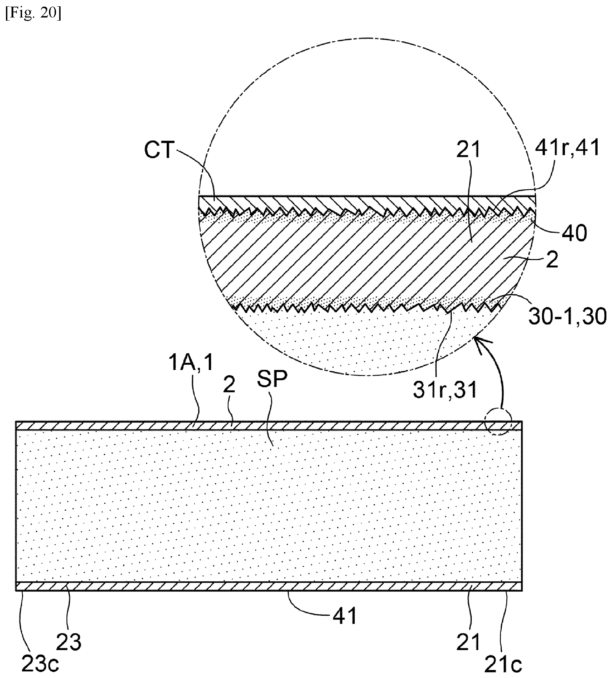

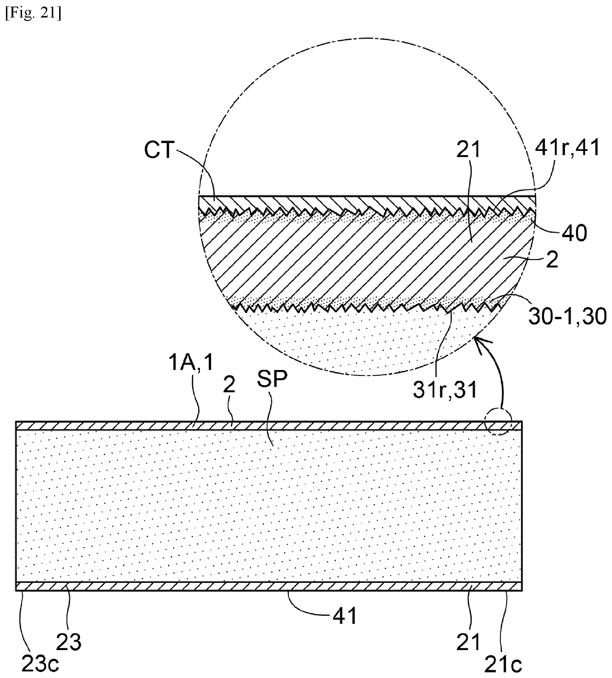

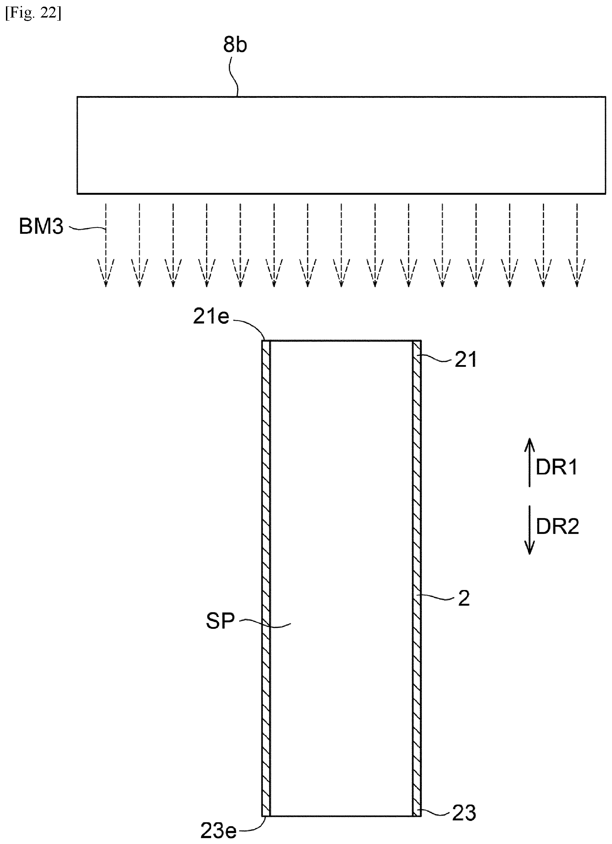

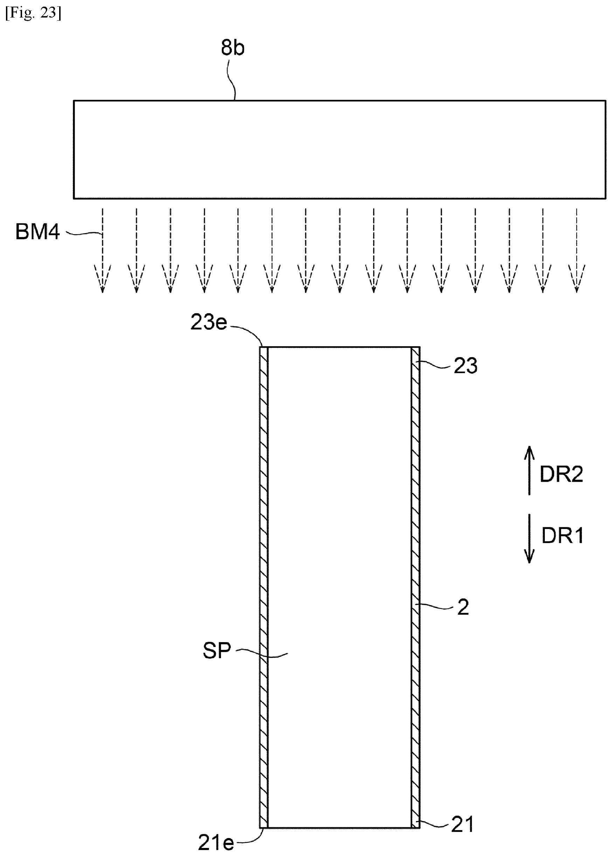

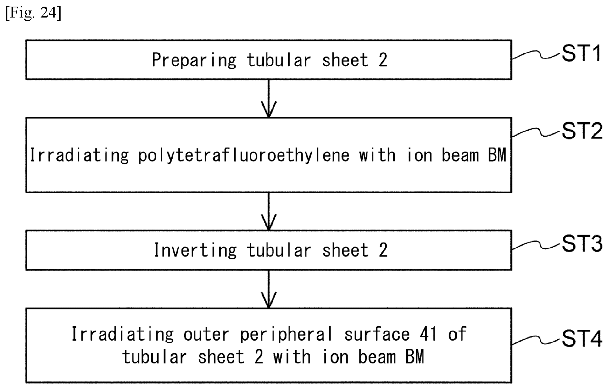

A method for manufacturing an artificial blood vessel 1 according to a second embodiment will be described with reference to FIGS. 1 to 24. FIG. 13 is a schematic perspective view schematically illustrating an example of the tubular sheet 2 prepared in a preparation step. FIG. 14 is a schematic cross-sectional view schematically illustrating a manner in which an ion beam BM is emitted from an ion irradiation apparatus 8a onto an inner surface of the tubular sheet 2. FIG. 15 is a schematic cross-sectional view schematically illustrating a manner in which an ion beam BM is emitted from an ion irradiation apparatus 8b onto an outer surface 2t of the tubular sheet 2. FIG. 16 is a diagram schematically illustrating a manner in which the tubular sheet 2 is inverted. FIG. 17 is a schematic perspective view schematically illustrating a state during execution of an inversion step. FIG. 18 is a schematic cross-sectional view schematically illustrating a state during execution of a second irradiation step. FIG. 19 is a schematic cross-sectional view schematically illustrating a manner in which an ion beam BM2 is emitted onto only a part of the outer peripheral surface 41 of the tubular sheet 2. FIG. 20 is a schematic cross-sectional view schematically illustrating a state in which, after execution of the second irradiation step, a biological adhesive J has been added to a surface of the second ion-implanted layer 40. FIG. 21 is a schematic cross-sectional view schematically illustrating a state in which, after execution of the second irradiation step, a coating CT for preventing leakage of blood has been added to a surface of the second ion-implanted layer 40. FIG. 22 is a schematic cross-sectional view schematically illustrating a manner in which an ion beam BM3 is emitted onto the end surface 21e of the tubular sheet 2 on the first direction DR1 side. FIG. 23 is a schematic cross-sectional view schematically illustrating a manner in which an ion beam BM4 is emitted onto the end surface 23e of the tubular sheet 2 on the second direction DR2 side. FIG. 24 is a flowchart illustrating an example of the method for manufacturing the artificial blood vessel 1 according to the second embodiment.

In the second embodiment, differences from the first embodiment will be mainly described. On the other hand, in the second embodiment, descriptions that would repeat matters already described in the first embodiment will be omitted. Therefore, it goes without saying that matters already described in the first embodiment can be applied to the second embodiment even if such matters are not explicitly described in the second embodiment. Conversely, matters described in the second embodiment can be employed in the first embodiment.

The artificial blood vessel 1 manufactured by the method for manufacturing an artificial blood vessel according to the second embodiment may be the artificial blood vessel 1A according to the first embodiment, or may be another artificial blood vessel.

As illustrated in FIG. 13, in a first step ST1, a tubular sheet 2 containing polytetrafluoroethylene is prepared. The first step ST1 is a preparation step.

As illustrated in FIG. 14, in a second step ST2, polytetrafluoroethylene is irradiated with an ion beam BM. The second step ST2 is an irradiation step. The irradiation step, that is, the second step ST2, includes irradiating the polytetrafluoroethylene with the ion beam BM such that an ion-implanted layer 30 is formed in the tubular sheet 2.

In the example illustrated in FIG. 14, the irradiation step, that is, the second step ST2, includes irradiating the inner surface of the tubular sheet 2 with the ion beam BM. By irradiating the inner surface of the tubular sheet 2 with the ion beam BM, the inner surface of the tubular sheet 2 becomes the roughened surface 31r. In addition, by irradiating the inner surface of the tubular sheet 2 with the ion beam BM, the inner surface of the tubular sheet 2 becomes the inner peripheral surface 31 of the ion-implanted layer 30. See, for example, FIG. 2. In the example illustrated in FIG. 14, the ion irradiation apparatus 8a includes a magnet, for example, a superconducting magnet 81, that deflects the ion beam BM.

Since the inner diameter of the artificial blood vessel is small, it is extremely difficult to irradiate the inner surface of the tubular sheet 2 with the ion beam BM. However, if the technique described in JP H5-128993 A is applied to the manufacture of the artificial blood vessel 1, it is not impossible to irradiate the inner surface of the tubular sheet 2 with the ion beam BM.

Alternatively, as illustrated in FIGS. 15 and 16, after the irradiation step, that is, the second step ST2, is performed, the tubular sheet 2 may be inverted such that the outer surface 2t of the tubular sheet 2 becomes an inner surface. By the inversion, the inner surface of the tubular sheet 2 becomes the roughened surface 31r. In addition, by the inversion, the inner surface of the tubular sheet 2 becomes the inner peripheral surface 31 of the ion-implanted layer 30.

As illustrated in FIG. 1, FIG. 7, or FIG. 9, the tubular sheet 2 includes an ion-implanted layer 30 as an inner layer. A main component of the ion-implanted layer 30 is polytetrafluoroethylene. An inner peripheral surface 31 of the ion-implanted layer 30 is constituted by a roughened surface 31r containing polytetrafluoroethylene as a main component.

In the second embodiment, a method for manufacturing the artificial blood vessel 1 having the inner peripheral surface 31 that favorably affects blood flow is provided.

### Optional Additional Configurations

Next, optional additional configurations that can be employed in the method for manufacturing the artificial blood vessel 1 according to the second embodiment will be described.

As illustrated in FIG. 13, in the first step ST1, that is, the preparation step, the tubular sheet 2 containing polytetrafluoroethylene is prepared.

The tubular sheet 2 prepared in the preparation step, that is, the first step ST1, may contain polytetrafluoroethylene as a main component. In other words, polytetrafluoroethylene may account for 50 percent by weight or more of the entire material constituting the tubular sheet 2. Polytetrafluoroethylene may account for 70 percent by weight or more, 90 percent by weight or more, 95 percent by weight or more, or 99 percent by weight or more of the entire material constituting the tubular sheet 2. The tubular sheet 2 prepared in the preparation step, that is, the first step ST1, may be constituted by ePTEF. The tubular sheet 2 prepared in the preparation step, that is, the first step ST1, is preferably a sheet in which polytetrafluoroethylene is exposed at a surface. In the example illustrated in FIG. 13, polytetrafluoroethylene is exposed at an outer peripheral surface of the tubular sheet 2 prepared in the preparation step, that is, the first step ST1. In addition, polytetrafluoroethylene is exposed at an inner peripheral surface of the tubular sheet 2 prepared in the preparation step, that is, the first step ST1.

A film thickness of the tubular sheet 2 prepared in the preparation step, that is, the first step ST1, is, for example, 0.01 mm or more and 2 mm or less. The film thickness of the tubular sheet 2 may be 0.05 mm or more and 1.5 mm or less, 0.1 mm or more and 1.0 mm or less, or 0.1 mm or more and 0.5 mm or less.

In the example illustrated in FIG. 13, the tubular sheet 2 prepared in the preparation step, that is, the first step ST1, is a seamless tubular body. The tubular sheet 2 prepared in the preparation step, that is, the first step ST1, may be a tubular body formed by extrusion molding.

As illustrated in FIG. 14 or FIG. 15, in the second step ST2, that is, the irradiation step, the polytetrafluoroethylene is irradiated with an ion beam BM such that an ion-implanted layer 30 is formed in the tubular sheet 2. The ion beam BM with which the polytetrafluoroethylene is irradiated in the irradiation step, that is, the second step ST2, may include argon ions, neon ions, or other ions. In other words, an element ion-implanted into the polytetrafluoroethylene in the irradiation step, that is, the second step ST2, may be argon, neon, or another element.

As illustrated in FIG. 14, the irradiation step, that is, the second step ST2, may include irradiating an inner surface of the tubular sheet 2 with the ion beam BM such that an inner surface 2n of the tubular sheet 2 is roughened.

In the example illustrated in FIG. 14, an angle α between a direction parallel to the longitudinal direction of the tubular sheet 2 and a direction in which the ion beam BM is incident on the polytetrafluoroethylene is 45 degrees or more and 90 degrees or less. The angle α is preferably 60 degrees or more, 70 degrees or more, 80 degrees or more, or 85 degrees or more. When the angle α is 45 degrees or more, the topology of the roughened surface 31r becomes favorable. In contrast, in the technique described in Patent Literature 2, an angle between a direction parallel to the longitudinal direction of the tube and a traveling direction of the ion beam BM is 1.7 degrees, and ions cannot deeply enter an inner layer of the tube. In other words, in the technique described in Patent Literature 2, a roughened surface that favorably affects blood flow is not sufficiently formed. In addition, in the technique described in Patent Literature 2, asymmetry in the topology of the roughened surface becomes excessive, and there is a possibility that restrictions may arise on an attachment direction of the tube to a blood vessel.

As illustrated in FIG. 15, the irradiation step, that is, the second step ST2, may include irradiating the outer surface 2t of the tubular sheet 2 with the ion beam BM such that the outer surface 2t of the tubular sheet 2 is roughened. In other words, the step of irradiating the polytetrafluoroethylene with the ion beam BM may include irradiating the outer surface 2t of the tubular sheet 2 with the ion beam BM.

In the example illustrated in FIG. 15, an angle α between a direction parallel to the longitudinal direction of the tubular sheet 2 and a direction in which the ion beam BM is incident on the polytetrafluoroethylene is 45 degrees or more and 90 degrees or less. The angle α is preferably 60 degrees or more, 70 degrees or more, 80 degrees or more, or 85 degrees or more. When the angle α is 45 degrees or more, the topology of the roughened surface 31r becomes favorable.

The irradiation step, that is, the second step ST2, may be performed in a state in which the tubular sheet 2 is rotated about a first axis AX1. In other words, the ion irradiation apparatus 8b may irradiate the outer surface 2t of the tubular sheet 2 rotating about the first axis AX1 with the ion beam BM. Alternatively, during execution of the irradiation step, that is, the second step ST2, an ion irradiation unit of the ion irradiation apparatus 8b may revolve about the first axis AX1.

By performing the irradiation step, that is, the second step ST2, the ion-implanted layer 30, more specifically, a layer in which an ion-implanted element is mixed with polytetrafluoroethylene, is formed in the tubular sheet 2. After the irradiation step, that is, the second step ST2, is performed, a layer containing the drug K may be added to a surface of the ion-implanted layer 30. Since the drug K has already been described in the first embodiment, repeated descriptions of the drug K are omitted.

As illustrated in FIGS. 16 and 17, in a third step ST3, the tubular sheet 2 may be inverted. The third step ST3 is an inversion step. In the inversion step, that is, the third step ST3, the tubular sheet 2 is inverted such that the outer surface 2t of the tubular sheet 2 becomes an inner surface.
By the step of inverting the tubular sheet, the outer surface 2t of the tubular sheet 2 irradiated with the ion beam becomes the inner surface of the tubular sheet 2.

In the examples illustrated in FIGS. 16 and 17, by the step of inverting the tubular sheet, the ion-implanted layer 30 becomes an inner layer of the tubular sheet 2. In addition, the inner peripheral surface 31 of the ion-implanted layer 30 becomes the roughened surface 31r. Since the ion-implanted layer 30, the inner peripheral surface 31, and the roughened surface 31r have already been described in the first embodiment, repeated descriptions of the ion-implanted layer 30, the inner peripheral surface 31, and the roughened surface 31r are omitted.

When the method for manufacturing the artificial blood vessel 1 includes the above-described inversion step, that is, the third step ST3, technical difficulty in irradiating the inner surface of the tubular sheet 2 with the ion beam BM can be avoided. Accordingly, the above-described irradiation step, that is, the second step ST2, can be performed using a more general ion irradiation apparatus.

As illustrated in FIG. 18, in a fourth step ST4, the outer peripheral surface 41 of the tubular sheet 2 may be irradiated with an ion beam BM2. The fourth step ST4 is a second irradiation step. The second irradiation step, that is, the fourth step ST4, includes irradiating the outer peripheral surface 41 of the tubular sheet 2 with the ion beam BM2 such that a second ion-implanted layer 40 is formed in the tubular sheet 2. By irradiating the outer peripheral surface 41 of the tubular sheet 2 with the ion beam BM2, at least a part of the outer peripheral surface 41 of the tubular sheet 2 becomes the second roughened surface 41r. See FIG. 8 or FIG. 10, if necessary. The second roughened surface 41r is constituted by a surface of the second ion-implanted layer 40.

The ion beam BM2 with which the outer peripheral surface 41 of the tubular sheet 2 is irradiated in the second irradiation step, that is, the fourth step ST4, may include argon ions, neon ions, or other ions. In other words, an element implanted into the tubular sheet 2 in the second irradiation step, that is, the fourth step ST4, more specifically, an element ion-implanted into the polytetrafluoroethylene, may be argon, neon, or another element.

The second irradiation step, that is, the fourth step ST4, may include irradiating the entirety of the outer peripheral surface 41 of the tubular sheet 2 with the ion beam BM2.

Alternatively, as illustrated in FIG. 19, the second irradiation step, that is, the fourth step ST4, may include irradiating only a part of the outer peripheral surface 41 of the tubular sheet 2 with the ion beam BM2. In the example illustrated in FIG. 19, the second irradiation step, that is, the fourth step ST4, includes irradiating a first region RG1 of the outer peripheral surface 41 of the tubular sheet 2 with the ion beam BM2. In the example illustrated in FIG. 19, the second irradiation step, that is, the fourth step ST4, is performed in a state in which a mask MK is disposed in a region facing a second region RG2 such that the ion beam BM2 does not reach the second region RG2 of the outer peripheral surface 41 of the tubular sheet 2.

In the example illustrated in FIG. 19, after the second irradiation step, that is, the fourth step ST4, is performed, the outer peripheral surface 411 of the first end portion 21 of the tubular sheet 2 becomes the roughened surface 411r, more specifically, a surface of the second ion-implanted layer 40.

In the example illustrated in FIG. 19, after the second irradiation step, that is, the fourth step ST4, is performed, the outer peripheral surface 413 of the second end portion 23 of the tubular sheet 2 becomes the roughened surface 413r, more specifically, a surface of the second ion-implanted layer 40.

In the example illustrated in FIG. 19, after the second irradiation step, that is, the fourth step ST4, is performed, the outer peripheral surface 415 of the longitudinal central region 25 of the tubular sheet 2 is a non-roughened surface. In other words, after the second irradiation step, that is, the fourth step ST4, is performed, the outer peripheral surface 415 of the longitudinal central region 25 of the tubular sheet 2 is maintained in a non-roughened state.

In the example illustrated in FIG. 18 or FIG. 19, the second irradiation step, that is, the fourth step ST4, may be performed in a state in which the tubular sheet 2 is rotated about a second axis AX2. In other words, the ion irradiation apparatus 8b may irradiate the outer peripheral surface 41 of the tubular sheet 2 rotating about the second axis AX2 with the ion beam BM2. Alternatively, during execution of the second irradiation step, that is, the fourth step ST4, an ion irradiation unit of the ion irradiation apparatus 8b may revolve about the second axis AX2. The second axis AX2 may be the same as the first axis AX1, or may be different from the first axis AX1.

The second irradiation step, that is, the fourth step ST4, illustrated in FIG. 18 or FIG. 19 may be performed after the irradiation step, that is, the second step ST2, illustrated in FIG. 14 is performed. The second irradiation step, that is, the fourth step ST4, illustrated in FIG. 18 or FIG. 19 may be performed before the irradiation step, that is, the second step ST2, illustrated in FIG. 14 is performed. Further alternatively, the second irradiation step, that is, the fourth step ST4, illustrated in FIG. 18 or FIG. 19 may be performed after the inversion step, that is, the third step ST3, illustrated in FIG. 16 is performed.

As illustrated in FIG. 20, after the second irradiation step, that is, the fourth step ST4, is performed, a biological adhesive J may be added to a surface of the second ion-implanted layer 40. Alternatively or additionally, as illustrated in FIG. 21, after the second irradiation step, that is, the fourth step ST4, is performed, a coating CT for preventing leakage of blood, for example, a layer of a biocompatible polymer material for preventing leakage of blood, may be added to the surface of the second ion-implanted layer 40. The coating CT, more specifically, the layer of the biocompatible polymer material, includes, for example, a gel polymer, more specifically, a biopolymer such as collagen and/or a biocompatible polymer such as polyethylene glycol. In the example illustrated in FIG. 21, since the coating CT, for example, the coating CT containing collagen or the coating CT containing polyethylene glycol, is added to the surface of the second ion-implanted layer 40, more specifically, the surface of the second ion-implanted layer 40 containing polytetrafluoroethylene as a main component, adhesion between the second roughened surface 41r, which is the surface of the second ion-implanted layer 40, and the coating CT is favorable.

The feature of adding the coating CT, for example, the coating CT containing collagen or the coating CT containing polyethylene glycol, to the surface of the second ion-implanted layer 40, more specifically, the surface of the second ion-implanted layer 40 containing polytetrafluoroethylene as a main component, can also be employed in the artificial blood vessel 1A according to the above-described first embodiment. In the first embodiment, the ion-implanted layer 30 is, for example, non-gel-like. In the first embodiment, a gel-like coating layer, for example, a gel-like coating layer containing collagen or a gel-like coating layer containing polyethylene glycol, may be added to a surface of the ion-implanted layer 30, more specifically, a surface of the non-gel-like ion-implanted layer 30. In addition, in the first embodiment, the second ion-implanted layer 40 is, for example, non-gel-like. In the first embodiment, a gel-like coating layer, for example, a gel-like coating layer containing collagen or a gel-like coating layer containing polyethylene glycol, may be added to a surface of the second ion-implanted layer 40, more specifically, a surface of the non-gel-like second ion-implanted layer 40.

In this case, the vascular patch applied to a blood vessel has, for example, the following configuration. (1) The vascular patch includes a sheet that covers a damaged site of a blood vessel wall, for example, a hole portion of the blood vessel wall. (2) The sheet includes an ion-implanted layer. The ion-implanted layer is, for example, non-gel-like. (3) A main component of the ion-implanted layer is polytetrafluoroethylene. (4) A surface of the ion-implanted layer is constituted by a roughened surface containing polytetrafluoroethylene as a main component. (5) A coating of a biocompatible polymer material that is to come into contact with the blood vessel wall, for example, a coating of a gel-like polymer material, more specifically, a coating containing collagen and/or polyethylene glycol, is added to the surface of the ion-implanted layer. The coating of the biocompatible polymer material, for example, the coating of the gel-like polymer material, may be configured to come into contact with blood flow, in other words, blood flowing through the blood vessel, via the damaged site of the blood vessel wall, for example, the hole portion of the blood vessel wall. Since adhesion between polytetrafluoroethylene and the biocompatible polymer material, for example, collagen and/or polyethylene glycol, is improved by the roughened surface, which is the surface of the ion-implanted layer, separation of the biocompatible polymer material that comes into contact with blood flow from the polytetrafluoroethylene is suppressed.
The biocompatible polymer material may elute into the blood flow. Elution is different from separation. In addition, the biocompatible polymer material may be biodegradable. Addition of the coating is performed after ion implantation into polytetrafluoroethylene is performed. In other words, after the ion-implanted layer is formed in the sheet, the coating is added to the surface of the ion-implanted layer. Since the ion-implanted layer and the roughened surface have already been described in the first embodiment, repeated descriptions of the ion-implanted layer and the roughened surface are omitted.

As illustrated in FIG. 22, the method for manufacturing the artificial blood vessel 1 according to the second embodiment may include a step of irradiating the end surface 21e of the tubular sheet 2 on the first direction DR1 side with an ion beam BM3. By irradiating the end surface 21e of the tubular sheet 2 on the first direction DR1 side with the ion beam BM3, the end surface 21e of the tubular sheet 2 on the first direction DR1 side becomes the third roughened surface 21r. The third roughened surface 21r is constituted by a surface of the third ion-implanted layer 20.

As illustrated in FIG. 23, the method for manufacturing the artificial blood vessel 1 according to the second embodiment may include a step of irradiating the end surface 23e of the tubular sheet 2 on the second direction DR2 side with an ion beam BM4. By irradiating the end surface 23e of the tubular sheet 2 on the second direction DR2 side with the ion beam BM4, the end surface 23e of the tubular sheet 2 on the second direction DR2 side becomes the fourth roughened surface 23r.

In the first embodiment, excluding the section describing "Drug," the terms "blood," "first blood vessel," "second blood vessel," and "artificial blood vessel" may be replaced with "body fluid," "first tubular tissue," "second tubular tissue," and "tubular prosthesis," respectively.

For example, another embodiment relates to the following tubular prosthesis.
(1) A tubular prosthesis comprising a tubular sheet defining a space through which body fluid flows,
   wherein the tubular sheet includes an ion-implanted layer as an inner layer,
   a main component of the ion-implanted layer is polytetrafluoroethylene, and
   an inner peripheral surface of the ion-implanted layer is constituted by a roughened surface containing the polytetrafluoroethylene as a main component.
(2) The tubular prosthesis according to (1),
   wherein the ion-implanted layer is constituted by a single layer in which the polytetrafluoroethylene is mixed with an element implanted by ion implantation.
(3) The tubular prosthesis according to (1) or (2),
   wherein at least a part of the roughened surface containing the polytetrafluoroethylene as a main component is exposed to the space through which the body fluid flows.
(4) The tubular prosthesis according to any one of (1) to (3),
   wherein the tubular sheet is seamless.
(5) The tubular prosthesis according to any one of (1) to (4),
   wherein at least a part of an outer peripheral surface of the tubular sheet is a second roughened surface.
(6) The tubular prosthesis according to (5),
   wherein at least a part of the outer peripheral surface of the tubular sheet is a non-roughened surface.
(7) The tubular prosthesis according to any one of (1) to (6),
   wherein, when a direction from a second end portion of the tubular sheet toward a first end portion of the tubular sheet is defined as a first direction, an end surface of the tubular sheet on the first direction side is a third roughened surface.

In the first embodiment, the terms "blood," "first blood vessel," "second blood vessel," and "artificial blood vessel" may be replaced with "lymph fluid," "first tubular tissue," "second tubular tissue," and "artificial lymphatic vessel," respectively. In the first embodiment, the terms "blood," "first blood vessel," "second blood vessel," and "artificial blood vessel" may be replaced with "digestive fluid," "first tubular tissue," "second tubular tissue," and "artificial digestive tract," respectively. In the first embodiment, the terms "blood," "first blood vessel," "second blood vessel," and "artificial blood vessel" may be replaced with "urine," "first tubular tissue," "second tubular tissue," and "artificial ureter," respectively.

In the second embodiment, the terms "blood" and "artificial blood vessel" may be replaced with "body fluid" and "tubular prosthesis," respectively.

For example, another embodiment relates to the following method for manufacturing a tubular prosthesis.
(1) A method for manufacturing a tubular prosthesis, the method comprising:
   preparing a tubular sheet containing polytetrafluoroethylene; and
   irradiating the polytetrafluoroethylene with an ion beam such that an ion-implanted layer is formed in the tubular sheet,
   wherein the tubular sheet includes the ion-implanted layer as an inner layer,
   a main component of the ion-implanted layer is the polytetrafluoroethylene, and
   an inner peripheral surface of the ion-implanted layer is constituted by a roughened surface containing the polytetrafluoroethylene as a main component.
(2) The method for manufacturing a tubular prosthesis according to (1),
   wherein an angle between a direction parallel to a longitudinal direction of the tubular sheet and a direction in which the ion beam is incident on the polytetrafluoroethylene is 45 degrees or more.
(3) The method for manufacturing a tubular prosthesis according to (1) or (2), the method comprising:
   inverting the tubular sheet such that an outer surface of the tubular sheet becomes an inner surface,
   wherein irradiating the polytetrafluoroethylene with the ion beam includes irradiating the outer surface of the tubular sheet with the ion beam, and
   by the step of inverting the tubular sheet, the outer surface irradiated with the ion beam becomes the inner surface.
(4) The method for manufacturing a tubular prosthesis according to any one of (1) to (3), the method comprising:
   irradiating an outer peripheral surface of the tubular sheet with an ion beam such that a second ion-implanted layer is formed in the tubular sheet,
   wherein, after the step of irradiating the outer peripheral surface of the tubular sheet with the ion beam is performed, a biological adhesive is added to a surface of the second ion-implanted layer.

In the second embodiment, the terms "blood" and "artificial blood vessel" may be replaced with "lymph fluid" and "artificial lymphatic vessel," respectively. In the second embodiment, the terms "blood" and "artificial blood vessel" may be replaced with "digestive fluid" and "artificial digestive tract," respectively. In the second embodiment, the terms "blood" and "artificial blood vessel" may be replaced with "urine" and "artificial ureter," respectively.

The present invention is not limited to the above-described embodiments or modifications, and it is apparent that the embodiments or modifications can be appropriately modified or changed within the scope of the technical concept of the present invention. In addition, various techniques used in the embodiments or modifications can also be applied to other embodiments or other modifications, as long as no technical contradiction arises. Furthermore, optional additional configurations in the embodiments or modifications can be omitted as appropriate.

### Reference Signs List

1, 1A: artificial blood vessel
2: tubular sheet
2n: inner surface
2t: outer surface
8a, 8b: ion irradiation apparatus
9: blood vessel
9a: first blood vessel
9b: second blood vessel
20: ion-implanted layer
21: first end portion
21c: first contact layer
21e: end surface
21r: roughened surface
23: second end portion
23c: second contact layer
23e: end surface
23r: roughened surface
25: longitudinal central region
30: ion-implanted layer
30d: recess
31: inner peripheral surface
31r: roughened surface
40: ion-implanted layer
41: outer peripheral surface
41r: roughened surface
41s: non-roughened surface
81: superconducting magnet
90: tissue
311: inner peripheral surface
311r: roughened surface
411: outer peripheral surface
411r: roughened surface
413: outer peripheral surface
413r: roughened surface
415: outer peripheral surface
415r: roughened surface
AX1: first axis
AX2: second axis
BM, BM2, BM3, BM4: ion beam
CT: coating
DR1: first direction
DR2: second direction
J: biological adhesive
K: drug
K1: blood coagulation inhibitor
K2: drug that regulates extension of vascular endothelial cells
MK: mask
PT: living tissue
RG1: first region
RG2: second region
SP: space

## Claims

1. An artificial blood vessel comprising a tubular sheet defining a space through which blood flows,
wherein the tubular sheet includes an ion-implanted layer as an inner layer,
a main component of the ion-implanted layer is polytetrafluoroethylene, and
an inner peripheral surface of the ion-implanted layer is constituted by a roughened surface containing the polytetrafluoroethylene as a main component.

2. The artificial blood vessel according to claim 1,
wherein the ion-implanted layer is constituted by a single layer in which the polytetrafluoroethylene is mixed with an element implanted by ion implantation.

3. The artificial blood vessel according to claim 1,
wherein at least a part of the roughened surface containing the polytetrafluoroethylene as a main component is exposed to the space through which the blood flows.

4. The artificial blood vessel according to any one of claims 1 to 3,
wherein the tubular sheet is seamless.

5. The artificial blood vessel according to any one of claims 1 to 3,
wherein at least a part of an outer peripheral surface of the tubular sheet is a second roughened surface.

6. The artificial blood vessel according to claim 5,
wherein at least a part of the outer peripheral surface of the tubular sheet is a non-roughened surface.

7. The artificial blood vessel according to any one of claims 1 to 3,
wherein a drug is supported on the inner peripheral surface of the ion-implanted layer.

8. The artificial blood vessel according to claim 7,
wherein the drug includes a blood coagulation inhibitor.

9. A method for manufacturing an artificial blood vessel, the method comprising:
preparing a tubular sheet containing polytetrafluoroethylene; and
irradiating the polytetrafluoroethylene with an ion beam such that an ion-implanted layer is formed in the tubular sheet,
wherein the tubular sheet includes the ion-implanted layer as an inner layer,
a main component of the ion-implanted layer is the polytetrafluoroethylene, and
an inner peripheral surface of the ion-implanted layer is constituted by a roughened surface containing the polytetrafluoroethylene as a main component.

10. The method for manufacturing an artificial blood vessel according to claim 9,
wherein an angle between a direction parallel to a longitudinal direction of the tubular sheet and a direction in which the ion beam is incident on the polytetrafluoroethylene is 45 degrees or more.

11. The method for manufacturing an artificial blood vessel according to claim 9 or 10, the method comprising:
inverting the tubular sheet such that an outer surface of the tubular sheet becomes an inner surface,
wherein the step of irradiating the polytetrafluoroethylene with the ion beam includes irradiating the outer surface of the tubular sheet with the ion beam, and
by the step of inverting the tubular sheet, the outer surface irradiated with the ion beam becomes the inner surface.

12. The method for manufacturing an artificial blood vessel according to claim 9 or 10,
wherein, after the step of irradiating the polytetrafluoroethylene with the ion beam is performed, a layer containing a drug is added to a surface of the ion-implanted layer.

13. The method for manufacturing an artificial blood vessel according to claim 9 or 10, the method comprising:
irradiating an outer peripheral surface of the tubular sheet with an ion beam such that a second ion-implanted layer is formed in the tubular sheet,
wherein, after the step of irradiating the outer peripheral surface of the tubular sheet with the ion beam is performed, at least one of a biological adhesive and a coating for preventing leakage of blood is added to a surface of the second ion-implanted layer.
